# EUROPEAN PATENT SPECIFICATION

(11) **EP 1 645 330 B1**
(45) Date of publication and mention of the grant of the patent: **17.12.2008**
(21) Application number: 05021327.1
(22) Date of filing: 29.09.2005
(51) Int. Cl.: B01J 19/00, B01L 3/00

(54) **Functional particle array and method of use thereof**
Array mit funktionellen Teilchen und Verfahren zu dessen Verwendung
Réseau avec des particules fonctionnelles et procédé d'utilisation de celui-ci

(30) Priority: 30.09.2004 JP 2004286446
(43) Date of publication of application: 12.04.2006
(73) Proprietor: HITACHI SOFTWARE ENGINEERING CO., LTD., Yokohama-shi, Kanagawa 230-0045 (JP)
(72) Inventor: Kogi, Osamu, c/o Hitachi Software, Shinagawa-ku Tokyo 140-0002 (JP)
(74) Representative: Liesegang, Roland

(56) References cited:
- WO-A-95/10344
- WO-A-99/60170
- WO-A-03/096015
- US-A1- 2002 072 065
- US-A1- 2003 215 862
- US-A1- 2004 229 346
- PATENT ABSTRACTS OF JAPAN vol. 2000, no. 15, 6 April 2001 (2001-04-06) -& JP 2000 346842 A (HITACHI LTD; HITACHI CHEM CO LTD), 15 December 2000 (2000-12-15)

## Description

### FIELD OF THE INVENTION

The present invention relates to a capillary bead array arranged with probe beads in a capillary formed on a soft resin or the like, and particularly to a functional particle array arranged with various functional particles together with the probe beads.

### BACKGROUND OF THE INVENTION

As conventional technology relating to a capillary bead array arranged with probe beads bound with probes for detection of target molecules that are biological substances in a capillary formed on a soft resin or the like, JP-A No. 346842/2000 is cited. In the above patent document, DNA hybridization reaction is explained as an example of the detection of target molecules.

In the above patent document, only three kinds of beads that are "probe beads" retaining probes, "dummy beads" not retaining probes, and "marker beads" to identify the order of each probe bead are disclosed as beads arrayed in a capillary.

In the above patent document, only the three kinds of beads, "probe beads", "dummy beads", and "marker beads", are disclosed, and there is no description on arraying other particles in a capillary.

In the capillary bead array disclosed in the above patent Document, there have been the following problems:
(1) When an agent or an enzyme that exerts a chemical effect on probe beads or target molecules captured on the probe beads is added into the capillary, not only should a solution present in the capillary be drained but also a solution containing the agent or the enzyme should be supplied.
(2) As to a sample to be added into the capillary, treatment of the sample before the addition to the capillary has not been specified. Therefore, it is not possible to separate and purify beforehand the target molecules according to various physicochemical properties of the target molecules (molecular size, charge, hydrophobicity, etc.) contained in the sample.
(3) The target molecules not captured by the probe beads in the capillary are drained outside the capillary as waste liquor after using the capillary bead array. Specifically, molecules harmful to organisms and environment are also drained.

EP 1 507 145 A1 discloses a functional particle array, wherein a plurality of DNA beads are arranged in a glass tube between mark beads.

### SUMMARY OF THE INVENTION

Objects of the present invention are to solve the above three problems by arraying various functional particles in a capillary for a capillary bead array and to provide a capillary bead array having various functions and high added value to users.

As a result of diligent study, the present inventors have arrived at the present invention upon discovering that the above problems are solved by arraying not only "probe beads", "dummy beads", and "marker beads" but also various functional particles in a capillary and making use of various functions of the functional particles.

According to one aspect of the present invention, a capillary bead array is provided with not only numerous probe beads bound with probes having a property of capturing target molecules on either the surface or inside or both thereof that are arrayed one-dimensionally or two-dimensionally in a capillary formed on a substrate but also at least one kind of functional particle that is arrayed together with the probe beads within the same capillary.

Here, the functional particles referred to in the present invention are :
microcapsules containing an agent or the like as a core substance;

According to another aspect of the present invention, a method of use of the above functional particle array includes the following modes:
(2) Microcapsules containing a solution of an agent, as a core substance, exerting a chemical effect on probe beads or target molecules captured on the probe beads are arrayed as at least one kind of functional particle together with the probe beads in the same capillary, and the agent is provided to the probe beads or the target molecules captured on the probe beads in the capillary by a method of rupturing the microcapsules containing the solution of the agent or by a method of releasing the solution of the agent slowly from the inside of the microcapsules.

Thus, specific functions of the functional particles of the present invention are as follows:
(1) An agent, enzyme, fluorescent labeling agent, or the like necessary for a chemical reaction performed in a capillary is stably retained in the capillary with the use of the functional particles, and the agent or the like is eluted in the capillary as needed.
(2) Various target molecules that are contained in a sample applied to a capillary are adsorbed on the functional particles and separated based on physicochemical properties of the target molecules, and the target molecules adsorbed on the functional particles are eluted as necessary.

In the capillary bead array according to the present invention, it is possible, in the first place, to retain stably in a capillary an agent, enzyme, fluorescent labeling agent, or the like necessary for a chemical reaction performed in the capillary with the use of the functional particles as well as to elute the agent or the like in the capillary as required. In the second place, it is possible to adsorb and separate various target molecules contained in a sample applied to the capillary on the functional particles based on physicochemical properties of the target molecules as well as to elute the target molecules adsorbed on the functional particles as required. According to the present invention, it is possible to provide a capillary bead array with high functions and offer significant added value to users.

### BRIEF DESCRIPTION OF THE DRAWINGS

Fig. 1A and 1B are conceptual diagrams of a functional particle array not forming part of the present invention arranged with an agent particle together with probe beads, where Fig. 1A is a conceptual diagram of a state before dissolving the agent particle, and Fig. 1B is a conceptual diagram of a state after dissolving the agent particle;
Fig. 2A to 2C are conceptual diagrams of a functional particle array arranged with a microcapsule containing a solution of an agent as a core substance together with probe beads, where Fig. 2A is a conceptual diagram of a state before releasing the agent from the microcapsule, Fig. 2B is a conceptual diagram of the microcapsule, and Fig. 2C is a conceptual diagram after releasing the agent from the microcapsule;
Fig. 3A to 3C are conceptual diagrams of still another functional particle array not forming part of the present invention arranged with a vesicle containing a solution of an agent as a core substance together with probe beads, where Fig. 3A is a conceptual diagram of a state before releasing the agent from the vesicle, Fig. 3B is a conceptual diagram of the vesicle, and Fig. 3C is a conceptual diagram after releasing the agent from the vesicle;
Fig. 4 is a conceptual diagram of still another functional particle array not forming part of the present invention arranged with an adsorption carrier particle together with probe beads;
Fig. 5 is a conceptual diagram of still another functional particle array not forming part of the present invention arranged with an ion exchange resin particle together with probe beads;
Fig. 6 is a conceptual diagram of still another functional particle array arranged with an hydrophobic particle together with probe beads; and
Fig. 7 is a conceptual diagram of still another functional particle array not forming part of the present invention arranged with a porous particle together with probe beads.

### DETAILED DESCRIPTION OF THE PREFERRED EMBODIMENTS

In the present invention, probe beads are made of plastic, glass, and the like and are spherical particles having a particle diameter of several micrometers to several tens of micrometers. Specific examples include polystyrene beads, polypropylene beads, magnetic beads, and the like, and fluorescence emission and the like on these beads can be detected by using a flow cytometer.

Further, in the present invention, the composition of a solution in a capillary is changed with the aim of releasing an agent from functional particles or absorbing and eluting a target molecule on and from the functional particles. Here, the main composition of the solution that can be changed includes
a) kind, concentration, and ionic strength of a salt,
b) hydrogen ion concentration (pH),
c) concentration of a surface active agent, and
d) concentration of a binding inhibitor for a target molecule.

Hereinafter, examples of the present invention are described.

### Example 1 (not forming part of the present invention) : Agent tablet

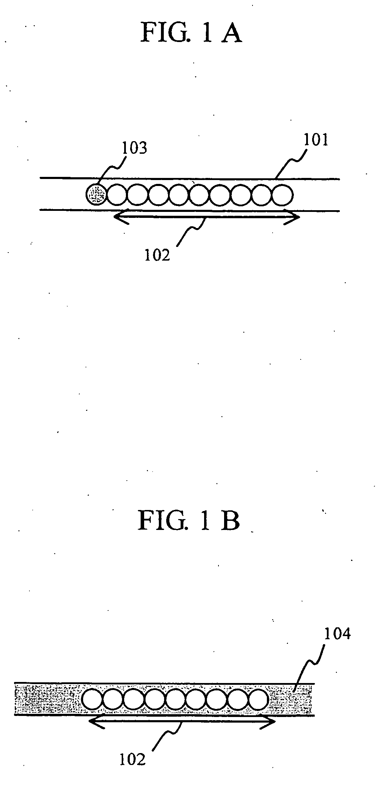

Fig. 1A and 1B show functional particle array arranged with a particle formed of an agent or the like in tablet that exerts a chemical effect on probe beads or target molecules captured by the probe beads and a method of use thereof.

When a solution having a temperature and composition corresponding to the solubility of the agent is added to the particle array (Fig. 1A) arranged with an agent particle 103 together with probe beads 102 in a capillary 101, the agent particle 103 is dissolved and eluted in the capillary 101, and the capillary is filled with a solution of the agent 104 (Fig. 1B). For example, when particles of two kinds of agents significantly different in solubility from each other are arrayed in a capillary, it is also possible to dissolve only the particle of one agent leaving the particle of the other agent undissolved by appropriately selecting the temperature and composition of the solution added into the capillary 101. In Fig. 1, only one piece of the agent particle 103 was arrayed for explanation, it is needless to say that the kind and the number of agent particles arrayable within the capillary are optional. Accordingly, it is possible to control the composition of the agent or the like eluted in the capillary and the timing of the elution by the combination of the agent particles arrayed in the capillary and the temperature and composition of the solution added into the capillary.

### Example 2: Microcapsule

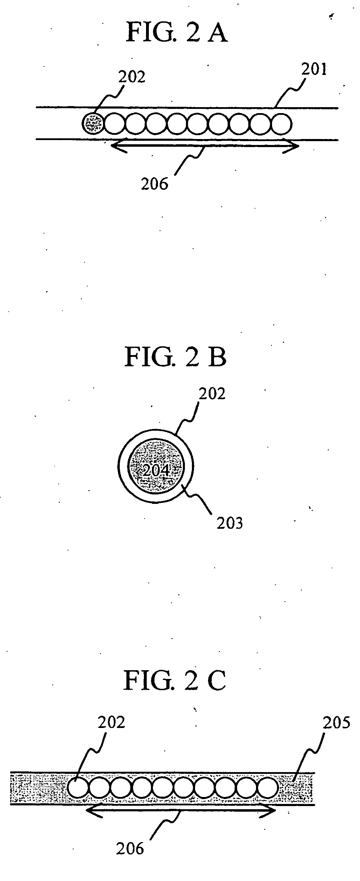

Fig. 2A to 2C show functional particle array arranged with a microcapsule containing a solution of an agent or the like that exerts a chemical effect on probe beads or target molecules captured by the probe beads as a core substance and a method of use thereof. Here, the microcapsule indicates an encapsulated particle containing a core substance within a particulate container formed of a capsule shell material. Physicochemical properties of the microcapsule widely vary depending on the kind of the capsule shell material and the kind of the core substance. Further, the microcapsule can be allowed to have a property to gradually release the core substance to the outside of the capsule shell material, that is, sustained-release. By adding a solution having a temperature and composition in accordance with the kind of a capsule shell material 203 of a microcapsule 202 and an agent 204 corresponding to the core substance into a capillary 201 (Fig. 2A) arrayed with the microcapsule 202 as well as probe beads 206, it is possible to induce the release of the agent 204 corresponding to the core substance and fill the capillary 201 with a solution of the agent 205 (Fig. 2C). Accordingly, it is possible to control the composition of the agent or the like eluted in the capillary and the timing of the elution by the combination of the kind of the microcapsule arrayed in the capillary and the temperature and composition of the solution added into the capillary.

### Example 3 (not forming part of the present invention) : Vesicle

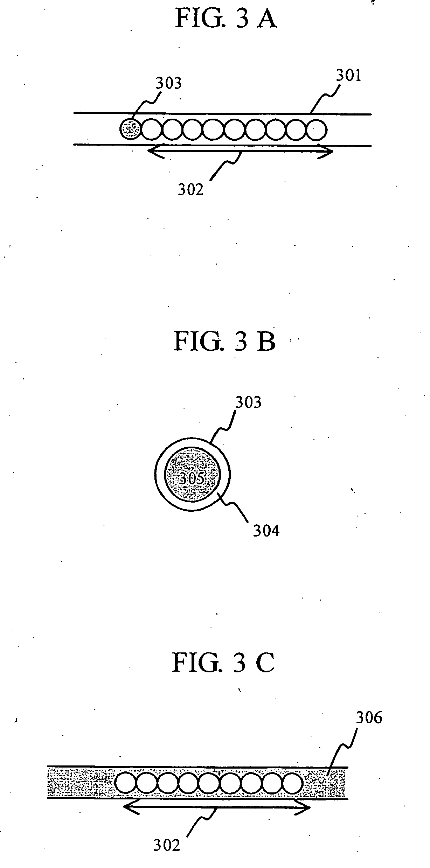

Fig. 3A to 3C show functional particle array arranged with a vesicle containing an agent or the like that exerts a chemical effect on probe beads or target molecules captured by the probe beads as a core substance and a method of use thereof. Here, the vesicle is an encapsulated particle prepared artificially by encapsulating a liquid that is the core substance in spherical particles formed of a membrane of an amphipathic substance such as lipid bilayer membrane. The core substance for the vesicle is in general an aqueous solution of an agent or an enzyme. The vesicle having an outer membrane formed of phospholipid such as lecithin is specifically called liposome. The vesicle is an artificially made encapsulated particle for the purpose of elucidating and mimicking various properties of biomembrane. The outer membrane shapes and physicochemical properties of the vesicle markedly differ depending on the kind and composition of the amphipathic substance forming the outer membrane and the temperature and composition of a solution surrounding the vesicle. Specifically, the core substance can be released to the outside of the outer membrane by providing physicochemical stimulation to the vesicle to perforate and rupture the outer membrane of the vesicle. Here, the physicochemical stimulation includes the following:
a) Change of the composition of a solution surrounding the vesicle (Here, the change of the composition of a solution indicates pH change and concentration change of a surface active agent of the solution as described above.)
b) Change of temperature
c) Change of pressure
d) Change of electric potential
e) Light irradiation

That is, when a vesicle 303 (Fig. 3B) together with probe beads 302 are arrayed in a capillary 301 (Fig. 3A) and physicochemical stimulation such as the above is given to the vesicle 303, an outer membrane 304 of the vesicle 303 is perforated or ruptured. As the result, an agent 305 corresponding to the core substance contained in the vesicle 303 is released, and the inside of the capillary 301 is filled with a solution of the agent 306 (Fig. 3C). Accordingly, it is possible to control the composition of the agent or the like released in the capillary and the timing of the release by the combination of the kind of vesicles arrayed in the capillary and the physicochemical stimulation given to the vesicle.

### Example 4 (not forming part of the present invention) : Adsorption carrier

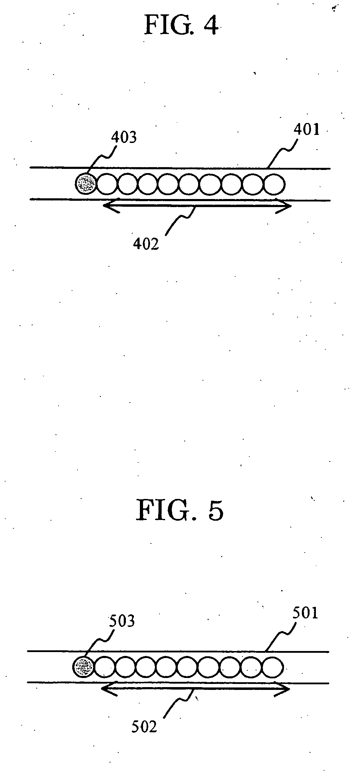

Fig. 4 shows a functional particle array arranged with an adsorption carrier particle having a large specific surface area and high adsorption ability and a method of use thereof. Here, the adsorption carrier indicates a porous inorganic substance and a porous polymer. Specifically, the porous inorganic substance indicates molecular sieve, activated charcoal, porous silica gel mainly composed of SiO₂, activated alumina mainly composed of aluminum oxide, and the like. A number of probe beads 402 are arrayed in a capillary 401. Among target molecules contained in a sample that was applied to the capillary 401, target molecules having affinity to probes on the probe beads 402 are bound to the probes to be captured on the probe beads 402.

In this case, when the adsorption carrier particle is not arrayed in the capillary 401, target molecules not captured on the probe beads 402 among the target molecules contained in the sample are discarded to the outside of the capillary as waste liquor after completion of the reaction with the capillary bead array regardless of whether the target molecules are biochemically or immunologically significant. On the other hand, when an adsorption carrier particle 403 is arrayed in the capillary 401, the target molecules not captured on the probe beads 402 among the target molecules contained in the sample can be adsorbed and recovered. Particularly, when the sample contains molecules that are harmful to living organisms and environment, these harmful molecules can be adsorbed on the adsorption carrier particle 403 and recovered. This leads to retaining the harmful molecules within the capillary after completion of the reaction with the capillary bead array, resulting in no discharge of the harmful molecules to the outside of the capillary. That is, advantages that not only is it prevented for a user to be exposed to harmful molecules after completion of the reaction with the capillary bead array but also a method of disposing of the harmful molecules is simplified can be offered.

### Example 5 (not forming part of the present invention) : Ion exchange resin

Fig. 5 shows a functional particle array arranged with an ion exchange resin particle having ion exchange ability and a method of use thereof. Ion exchange resins are broadly classified into cation exchange resin and anion exchange resin. The characteristic of an ion exchange resin lies in that a counter ionic substance can be captured on or eluted from the ion exchange resin by selecting a solution corresponding to a mobile phase in liquid column chromatography. A cationic substance in cation exchange resin and an anionic substance in anion exchange resin correspond to counter ionic substances, respectively.

An example of a cation exchange resin particle is explained in Fig. 5. A cation exchange resin particle 503 is arrayed together with probe beads 502 in a capillary 501. When a sample containing target molecules is applied to the capillary 501, cationic target molecules are selectively captured on the cation exchange resin particle 503. Accordingly, only anionic or neutral target molecules contained in the sample can be selectively analyzed on the probe beads 502. In addition, the cationic target molecules captured on the cationic ion exchange resin particle 503 can be eluted as needed. The conditions for eluting the cationic target molecules captured on the ion exchange resin are similar to those used for general ion exchange chromatography and are as follows:
a) Salt concentration of a solution in a capillary is varied with the used of NaCl, KCl, Na₂SO₄, and the like;
b) pH of the solution in the capillary is varied; and so forth.

Further, when a cationic agent was captured in advance on the cation exchange resin particle 503, it is possible to elute the cationic agent captured on the cation exchange resin particle 503 into a solution in the capillary 501 as needed. In this case, the cation exchange resin particle 503 acts as a particle to adsorb and elute the agent or the like in a way similar to the agent particle in the example 1, the microcapsule in the example 2, and the vesicle in the example 3.

In addition, it is needless to say that the function described in the example 5 can be realized for an anion exchange resin particle.

Thus, it is possible to capture counter ionic target molecules applied to a capillary as well as to elute the counter ionic target molecules as required by arraying an ion exchange resin particle in the capillary.

### Example 6 (not forming part of the present invention) : Hydrophobic particle

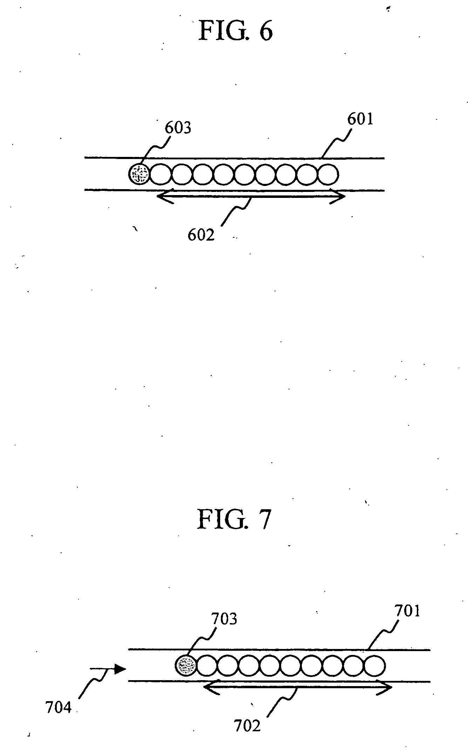

Fig. 6 shows a functional particle array arranged with a hydrophobic particle having a functional group with low polarity on its surface and high surface hydrophobicity and a method of use thereof. Specific examples of the hydrophobic particles include hydrophobic carriers that are commonly used in reversed phase chromatography and hydrophobic chromatography. Adsorption and elution of hydrophobic target molecules can be carried out on the hydrophobic particle by taking advantage of hydrophobic interaction between these hydrophobic particle and hydrophobic target molecules. That is, the hydrophobic interaction is strengthened in a hydrophilic solvent, and the hydrophobic target molecules are adsorbed on the hydrophobic particle. On the other hand, the hydrophobic interaction is weakened in a lipophilic solvent, and therefore, the hydrophobic target molecules tend to be eluted from the hydrophobic particle. Methods for adsorbing the target molecules on the hydrophobic particle and eluting the target molecules from the hydrophobic particle are broadly divided into the following two ways:
(1) A method in which the proportion of water contained in a solvent is changed
   This is a method for adsorbing and eluting target molecules in common reversed phase chromatography. First, the target molecules are adsorbed on hydrophobic particles in a solvent containing a larger proportion of water. Subsequently, the target molecules are eluted from the hydrophobic particles by lowering the proportion of water in the solvent to diminish the hydrophobic interaction.
(2) A method in which salting out is utilized
   This is a method for adsorbing and eluting target molecules in common hydrophobic chromatography. First, the target molecules are adsorbed on hydrophobic particles in a solvent of high ionic strength. Subsequently, the ionic strength of the solvent is lowered to elute the target molecules.

In the present invention, adsorption and elution of hydrophobic target molecules on hydrophobic particles arrayed in a capillary are carried out based on the above methods. A hydrophobic particle 603 is arrayed together with probe beads 602 in a capillary 601. A sample containing target molecules is applied to the capillary 601, and hydrophobic target molecules are selectively adsorbed on the hydrophobic particle 603. Accordingly, only hydrophilic target molecules contained in the sample can be selectively analyzed on the probe beads 602. Further, the hydrophobic target molecules captured on the hydrophobic particle 603 can be eluted as required.

Furthermore, when a hydrophobic agent or the like is captured in advance on the hydrophobic particle 603, it is possible to elute the hydrophobic agent adsorbed on the hydrophobic particle 603 in a solution in the capillary 601. In this case, the hydrophobic particle 603 acts as a particle for adsorption and elution of the agent or the like similarly to the agent particle in the example 1, the microcapsule in the example 2, and the vesicle in the example 3.

Accordingly, by arraying hydrophobic particles in a capillary, it is possible not only to adsorb hydrophobic target molecules applied to the capillary but also to elute the hydrophobic target molecules as needed.

### Example 7 (not forming part of the present invention) : Porous particle

Fig. 7 shows a functional particle array arranged with a porous particle having a number of pores of molecular size and a method of use thereof. A specific example of the porous particle can be a porous particle for use in common gel filtration chromatography. In gel filtration chromatography, when a sample containing target molecules is applied on a column packed with porous particles, target molecules smaller in apparent molecular size compared to pore sizes of the porous particles having numerous pores move so as to penetrate into the inside of the porous particles, while target molecules larger in apparent molecular size move along the outside of the porous particles. Since the pore sizes of the porous particles are not constant but variable with certain distribution, traveling distances of target molecules through the column differ depending on apparent molecular sizes of the target molecules, which results in differences in elution rates of the target molecules. In the present invention, target molecules different in molecular sizes are separated with the use of porous particles arrayed in a capillary based on the above principle.

A porous particle 703 is arrayed together with probe beads 702 in a capillary 701. Then, a sample containing target molecules having various molecular sizes is applied to the capillary. For explanation in Fig.7, the sample is applied from a direction of sample load 704. At this time, the target molecules contained in the sample are adsorbed on the porous particle 703. Then, elution starts from target molecules larger in molecular sizes, and target molecules smaller in molecular sizes are gradually eluted. Therefore, the target molecules contained in the sample can be analyzed on the probe beads 702 according to their molecular sizes. As described above, by arraying porous particles in a capillary, it is possible to separate and analyze target molecules applied to a capillary according to their molecular sizes.

In the capillary bead array according to the present invention, it is possible, in the first place, to retain stably in a capillary an agent, enzyme, fluorescent labeling agent, or the like necessary for a chemical reaction performed in the capillary with the use of the functional particles as well as to elute the agent or the like in the capillary as required. In the second place, it is possible to adsorb and separate various target molecules contained in a sample applied to the capillary on the functional particles based on physicochemical properties of the target molecules as well as to elute the target molecules as required.

By adding the foregoing functions to capillary bead array, the field of use of capillary bead array can be substantially expanded.

## Claims

1. A functional particle array for a capillary bead array that is arranged one-dimensionally or two-dimensionally with numerous probe beads bound with probes having a property of capturing target molecules on either the surface or inside or both thereof in a capillary formed on a substrate, said functional particle array comprising said capillary bead array and further comprising at least one kind of functional particle arrayed together with the probe beads in the same capillary, **characterised in that** the functional particles are microcapsules containing, as a core substance, a solution of an agent exerting a chemical effect on the probe beads or the target molecules captured on the probe beads.

2. The functional particle array according to claim 1, wherein the diameter of the functional particles is approximately equal to the diameter of the probe beads.

3. A method of use of a functional particle array according to any of claims 1 to 2 comprising:
arraying microcapsules containing a solution of an agent, as a core substance, exerting a chemical effect on probe beads or target molecules captured on the probe beads as at least one kind of functional particle together with the probe beads in the same capillary; and
providing the agent to the probe beads or the target molecules captured on the probe beads in the capillary by rupturing the microcapsules containing the solution of the agent.

4. The method of use of a functional particle array according to claim 3, wherein the agent is provided to the probe beads or the target molecules captured on the probe beads in the capillary by releasing the solution of the agent slowly from the inside of the microcapsules.

## Patentansprüche

1. Funktioneller Teilchen-Array für einen Kapillar-Kügelchen-Array ("capillary bead array"), der eindimensional oder zweidimensional angeordnet ist, wobei zahlreiche Sondenkügelchen mit Sonden gebunden sind, die die Eigenschaft haben, Zielmoleküle auf entweder der Oberfläche oder Innenseite oder beidem in einer Kapillare einzufangen, die auf einem Substrat gebildet ist, wobei der funktionelle Teilchen-Array den Kapillar-Kügelchen-Array umfasst und weiterhin wenigstens eine Art von funktionellen Teilchen umfasst, die zusammen mit den Sondenkügelchen in derselben Kapillare angeordnet sind, **dadurch gekennzeichnet, dass** die funktionellen Teilchen Mikrokapseln sind, die als eine Kemsubstanz eine Lösung eines Mittels enthalten, das einen chemischen Effekt auf die Sondenkügelchen oder die Zielmoleküle ausübt, die auf den Sondenkügelchen eingefangen sind.

2. Funktioneller Teilchen-Array nach Anspruch 1, wobei der Durchmesser der funktionellen Teilchen näherungsweise gleich dem Durchmesser der Sondenkügelchen ist.

3. Verfahren zur Verwendung eines funktionellen Teilchen-Array nach einem der Ansprüche 1 bis 2, umfassend:
Anordnen von Mikrokapseln, enthaltend eine Lösung eines Mittels als eine Kemsubstanz, die einen chemischen Effekt auf Sondenkügelchen oder Zielmoleküle ausübt, die auf den Sondenkügelchen eingefangen sind, als wenigstens eine Art von funktionellen Teilchen zusammen mit den Sondenkügelchen in derselben Kapillare; und
Bereitstellen des Mittels für die Sondenkügelchen oder die auf den Sondenkügelchen in der Kapillare eingefangenen Zielmoleküle durch Aufbrechen der Mikrokapseln, die die Lösung des Mittels enthalten.

4. Verfahren zur Verwendung eines funktionellen Teilchen-Array nach Anspruch 3, wobei das Mittel für die Sondenkügelchen oder die auf den Sondenkügelchen in der Kapillare eingefangenen Zielmoleküle bereitgestellt wird, indem die Lösung des Mittels langsam vom Inneren der Mikrokapseln freigesetzt wird.

## Revendications

1. Réseau de particules fonctionnelles pour un réseau de perles capillaire qui est agencé de façon unidimensionnelle ou bidimensionnelle avec de nombreuses perles sondes liées à des sondes ayant pour propriété de capturer des molécules cibles sur leur surface ou à l'intérieur ou les deux, dans un capillaire formé sur un substrat, le réseau de particules fonctionnelles comprenant le réseau de perles capillaire et comprenant en outre au moins une sorte de particules fonctionnelles disposées en réseau avec les perles sondes dans le même capillaire, **caractérisé en ce que** les particules fonctionnelles sont des micro-capsules contenant, en tant que substance de coeur, une solution d'un agent exerçant un effet chimique sur les perles sondes ou sur les molécules cibles capturées sur les perles sondes.

2. Réseau de particules fonctionnelles selon la revendication 1, dans lequel le diamètre des particules fonctionnelles est approximativement égal au diamètre des perles sondes.

3. Procédé d'utilisation d'un réseau de particules fonctionnelles selon l'une quelconque des revendications 1 à 2, comprenant :
disposer en réseau des micro-capsules contenant une solution d'un agent, en tant que substance de coeur, exerçant un effet chimique sur des perles sondes ou des molécules cibles capturées sur les perles sondes en tant qu'au moins une sorte de particules fonctionnelles avec les perles sondes dans le même capillaire ; et
fournir ledit agent aux perles sondes ou aux molécules cibles capturées sur les perles sondes dans le capillaire en brisant les micro-capsules contenant la solution dudit agent.

4. Procédé d'utilisation d'un réseau de particules fonctionnelles selon la revendication 3, dans lequel l'agent est fourni aux perles sondes ou aux molécules cibles capturées sur les perles sondes dans le capillaire en libérant la solution contenant l'agent lentement à partir de l'intérieur des micro-capsules.
